# EUROPÄISCHE PATENTSCHRIFT

(11) **EP 3 546 895 B1**
(45) Veröffentlichungstag und Bekanntmachung des Hinweises auf die Patenterteilung: **27.05.2020**
(21) Anmeldenummer: 18164600.1
(22) Anmeldetag: 28.03.2018
(51) Int. Cl.: G01D 11/24

(54) **VORRICHTUNG UND VERFAHREN ZUM BETREIBEN EINES BEHEIZBAREN SENSORS, INSBESONDERE IN EINER EXPLOSIONSFÄHIGEN ATMOSPHÄRE**
DEVICE AND METHOD FOR OPERATING A HEATABLE SENSOR, IN PARTICULAR IN AN EXPLOSIVE ATMOSPHERE
DISPOSITIF ET PROCÉDÉ DE FONCTIONNEMENT D'UN CAPTEUR CHAUFFANT, EN PARTICULIER DANS UNE ATMOSPHÈRE EXPLOSIVE

(43) Veröffentlichungstag der Anmeldung: 02.10.2019
(73) Patentinhaber: E+E ELEKTRONIK GES.M.B.H., 4209 Engerwitzdorf (AT)
(72) Erfinder: Birklbauer, Martin, 4240 Freistadt (AT); Jahn, Karl, 4623 Gunskirchen (AT); Schinnerl, Mario, 4224 Wartberg/Aist (AT)
(74) Vertreter: Maikowski & Ninnemann Patentanwälte Partnerschaft mbB

(56) Entgegenhaltungen:
- DE-A1- 4 026 762
- JP-A- 2007 155 639
- US-A- 5 121 627
- US-A1- 2006 162 426
- US-B1- 6 469 303

## Beschreibung

Die vorliegende Erfindung betrifft eine Kapselvorrichtung für einen Sensor. Sie betrifft ferner ein Sensorsystem und ein Verfahren zum Betreiben eines beheizbaren Sensors.

Viele Sensorarten, darunter Feuchtigkeits-, Taupunkt-, CO2- oder Sauerstoffsensoren, werden zu ihrem Betrieb mit elektrischer Leistung beaufschlagt. Als Folge davon ist die Temperatur an einer Oberfläche des Sensors typischerweise gegenüber einer Umgebungstemperatur erhöht. Die Wärmebildung ergibt sich dabei beispielsweise als Begleiteffekt, wie durch das Betreiben einer Lampe im Fall von CO2-Sensoren, oder als ein Erfordernis der Sensorfunktion selbst sein, wie im Fall einiger Feuchtigkeitssensoren. Sensoren, bei deren Betrieb Wärmebildung auftritt, werden vorliegend zusammenfassend als "beheizbare Sensoren" bezeichnet.

Bei der Verwendung beheizbarer Sensoren in explosionsgefährdeten Bereichen, etwa in einer explosionsfähigen Atmosphäre, kann sich die Gefahr einer ungewollten Zündung ergeben. Zu den Gefahrenquellen gehören dabei sowohl die erhitzte Oberfläche des Sensors als auch die Möglichkeit elektrischer Funkenbildung in einem Schaltkreis des Sensors. Dennoch besteht besonders in explosionsgefährdeten Bereichen oft ein Bedürfnis nach der Kenntnis von Messgrößen, für deren Erfassung beheizbare Sensoren erforderlich oder vorteilhaft sind.

Verschiedene Techniken sind bekannt, um eine Zündgefahr beim Betrieb von beheizbaren Sensoren in explosionsgefährdeten Bereichen zu verringern. Derzeit geltende Anforderungen an technische Geräte in Bezug auf Explosionsschutz sind zudem beispielsweise in der europäischen technischen Norm EN60079-0 und der entsprechenden internationalen Norm IEC60079-0 definiert.

Bei der Zündschutzart "Eigensicherheit" ist eine elektrische Leistungsaufnahme des Sensors oder anderer elektrischer Komponenten derart begrenzt, dass dabei keine gefährliche elektrische Funkenbildung und keine Oberflächentemperaturen oberhalb einer Grenzwerttemperatur auftreten können. Derzeit geltende Anforderungen an Eigensicherheit als Zündschutzart sind beispielsweise in der europäischen technischen Norm EN60079-11 und der entsprechenden internationalen Norm IEC60079-11 definiert. Eigensicherheit als Zündschutzart bedingt allgemein erhebliche Einschränkungen bezüglich der Gestaltung und des Betriebs von Sensoren.

Als Alternative erfordert die Zündschutzart "druckfeste Kapselung" den Einschluss des Sensors oder anderer elektrischer Komponenten in ein mechanisch stabiles Gehäuse. Das Gehäuse muss dabei so beschaffen sein, dass es einer darin gegebenenfalls stattfindenden Explosion standhält und keine explosionsgefährlichen Gase nach außen dringen lässt. Derzeit geltende Anforderungen an eine druckfeste Kapselung als Zündschutzart sind beispielsweise in der europäischen technischen Norm EN60079-1 und der entsprechenden internationalen Norm IEC60079-1 definiert. Im Geltungsbereich der vorgenannten Normen ist jedoch in einem Bereich mit zeitlich überwiegender explosionsfähiger Atmosphäre ("Zone 0") eine Verwendung beheizbarer Sensoren, die allein mit dieser Zündschutzart gesichert sind, nicht zulässig.

In der US 2006/0162426 ist ein Gassensor beschrieben, bei dem als Explosionsschutz ein Körper aus gesintertem Metall zwischen dem Messelement und einer Kappe aus porösem Kunststoff angeordnet ist.

Als weitere Alternative erfordert die Zündschutzart "Sandkapselung" ein Verfüllen des Sensors oder anderer elektrischer Komponenten mit einem Füllgut aus Sand oder Glasperlen. Kommt es dabei zu einer Zündung an der Sensoroberfläche, ist der Druckanstieg wegen der kleinen Zwischenräume im Füllgut vergleichsweise gering. Gleichzeitig kühlt das Füllgut die heißen Gase ab, sodass diese bei Austreten keine zündgefährlich hohe Temperatur mehr aufweisen. Derzeit geltende Anforderungen an eine Sandkapselung als Zündschutzart sind beispielsweise in der europäischen technischen Norm EN60079-5 und der entsprechenden internationalen Norm IEC60079-5 definiert. Im Geltungsbereich der vorgenannten Normen ist jedoch in einem Bereich mit zeitlich überwiegender explosionsfähiger Atmosphäre ("Zone 0") eine Verwendung beheizbarer Sensoren, die allein mit dieser Zündschutzart gesichert sind, ebenfalls nicht zulässig.

Aus der DE 82 25 525 U1 ist ein Sondenteil zur Messung von Gas bzw. Gas/Luftgemischen bekannt. Um gegenüber einer druckfesten Kapselung als Zündschutzart ein Eindringen des zu messenden Gases in das Sondengehäuse zu begünstigen, ist dabei die Sonde gemäß einer Sandkapselung als alternativer Zündschutzart in eine Schüttung aus Sand oder Glaskugeln gebettet. Abhängig vom Korndurchmesser des verwendeten Füllguts sind so größere Poren in der Gehäusewand und damit ein einfacherer Gasaustausch mit der Sondenumgebung möglich.

Bei der genannten Vorrichtung können sich Probleme daraus ergeben, dass die Sonde in das Füllgut eingebettet ist. Dies schließt eine Verwendung mit einigen kontaktempfindlichen Sonden aus. Zudem ist hierdurch ein Entfernen und Einsetzen von Sonden in das Gehäuse, beispielsweise zum Auswechseln oder Warten der Sonden, erschwert oder unmöglich. Weitere Probleme können sich daraus ergeben, dass bei der Verwendung mit einem beheizbaren Sensor ein Einsatz in einer explosionsfähigen Atmosphäre noch immer mit erheblicher Gefahr verbunden ist, etwa wenn die Leistungsaufnahme bzw. die Wärmeabgabe des Sensors entsprechende Normgrenzen, beispielsweise für eine Eigensicherheit als Zündschutzart, überschreitet. In einem solchen Fall würde zudem nur eine Zündschutzart Anwendung finden, was ebenfalls gängigen Redundanzanforderungen bezüglich eines Zündschutzes, etwa in Bezug auf einen "Zweifehlerfall", nicht genügen könnte.

In der US 5,121,627 ist außerdem ein Gas- und Dampfsensor beschrieben, bei dem um einen perforierten Versteifungsabschnitt eine gasdurchlässige Schicht aus nichtkompressiblem Material angeordnet ist, die wiederum als Unterlage für eine poröse Schicht aus Dimethyl-Silizium dient. In der DE 40 26 762 A1 ist eine Flammensperre mit einem Gehäuse beschrieben. Dabei füllt eine Schicht aus Glaskugeln oder Sand einen Innenraum des Gehäuses vollständig aus.

Somit stellt sich die Aufgabe, eine Technik vorzuschlagen, die das Betreiben eines beheizbaren Sensors insbesondere in einer explosionsfähigen Atmosphäre begünstigt.

Hiervon ausgehend werden vorliegend gemäß dem unabhängigen Anspruch 1 eine Kapselvorrichtung für einen Sensor, insbesondere zum Betreiben des Sensors in einer explosionsfähigen Atmosphäre, sowie gemäß dem unabhängigen Anspruch 11 ein Sensorsystem und gemäß dem unabhängigen Anspruch 15 ein Verfahren zum Betreiben eines beheizbaren Sensors vorgeschlagen. Merkmale einiger Ausführungsbeispiele sind in den Unteransprüchen angegeben. Die Merkmale der Unteransprüche können miteinander zur Ausbildung weiterer Ausführungsformen kombiniert werden, sofern nicht ausdrücklich etwas anderes angegeben ist.

Die Kapselvorrichtung umfasst ein Schutzgehäuse mit wenigstens einem gasdurchlässigen Wandbereich, der einen Gasaustausch zwischen einem Innenraum der Kapselvorrichtung und einer Umgebung der Kapselvorrichtung durch den gasdurchlässigen Wandbereich gestattet, und ein mit einem Füllgut befülltes Löschvolumen, das entlang einer Innenseite des Schutzgehäuses angeordnet ist und das einen zur Aufnahme eines Sensors vorgesehenen Aufnahmeraum der Kapselvorrichtung wenigstens teilweise umgibt. Die Kapselvorrichtung umfasst zudem ein gasdurchlässiges Filterelement, das zwischen dem Löschvolumen und dem Aufnahmeraum angeordnet ist und das Löschvolumen in Bezug auf den Aufnahmeraum begrenzt.

Das Filterelement kann dazu ausgebildet sein, ein Eindringen von Füllgut aus dem Löschvolumen in den Aufnahmeraum zu verhindern. Insbesondere kann das Filterelement dazu ausgebildet sein, ein Eindringen von wenigstens einem Teil des Füllguts, beispielsweise von jedwedem Teil des Füllguts, aus dem Löschvolumen in den Aufnahmeraum zu verhindern. Zusätzlich oder alternativ dazu kann das Filterelement dazu ausgebildet sein, einen mechanischen Kontakt zwischen dem Füllgut und/oder dem Filterelement und wenigstens einem Teil eines Sensors zu verhindern, wenn der Sensor in dem Aufnahmeraum angeordnet ist.

Das Filterelement kann starres Material, beispielsweise starren Kunststoff oder Metall, umfassen. Zusätzlich oder alternativ dazu kann das Filterelement flexibles Material, beispielsweise ein flexibles Kunststoff- und/oder Metallgewebe, umfassen. Insbesondere kann das Filterelement einen Drahtgitterfilter umfassen. Zudem kann eine kleinste Korngröße des Füllguts größer sein als eine größte Porengröße des Filterelements. Das Füllgut kann Sand und/oder Glasperlen umfassen.

Die Bereitstellung des Filterelements begünstigt eine Handhabung der Kapselvorrichtung getrennt von einem Sensor. Insbesondere ist mittels des Filterelements vermeidbar, dass Füllgut aus dem Löschvolumen dringt, wenn kein Sensor in dem Aufnahmeraum der Kapselvorrichtung angeordnet ist. Zudem gestattet das Filterelement ein Vermeiden eines mechanischen Kontakts zwischen einem kontaktempfindlichen Sensorelement des Sensors und dem Füllgut und/oder dem Filterelement.

Das Löschvolumen kann so beschaffen sein, dass es in Bezug auf den Sensor den Anforderungen an eine Sandkapselung als eine erste Zündschutzart entspricht. Die Anforderungen an die Sandkapselung als Zündschutzart können dabei durch die technische Norm EN60079-5 und/oder die technische Norm IEC60079-5 bestimmt sein. Das Schutzgehäuse kann so beschaffen sein, dass es in Bezug auf den Sensor den Anforderungen an eine druckfeste Kapselung als eine weitere Zündschutzart entspricht. Die Anforderungen an die druckfeste Kapselung als Zündschutzart können dabei durch die technische Norm EN60079-1 und/oder die technische Norm IEC60079-1 bestimmt sein.

Die Bereitstellung der Zündschutzarten einer Sandkapselung und einer druckfesten Kapselung kann eine Redundanz bezüglich der vorliegenden Zündschutzarten ermöglichen. Dabei kann eine solche Redundanz eine Verwendung der Kapselvorrichtung in Entsprechung mit technischen Sicherheitsvorschriften auch mit solchen beheizbaren Sensoren gestatten, deren Einsatz mit nur einer der Zündschutzarten gemäß denselben technischen Sicherheitsvorschriften nicht zulässig ist.

Der Sensor kann ein beheizbarer Sensor sein. Dabei kann der beheizbare Sensor einen Feuchtesensor, einen Taupunktsensor, einen CO2-Sensor und/oder einen Sauerstoffsensor umfassen. Insbesondere kann der beheizbare Sensor ein Taupunktmessgerät mit einem Feuchtesensor, der eine Ausheizfunktion aufweist, umfassen. Die Ausheizfunktion kann dazu vorgesehen sein, chemische Verunreinigungen auszuheizen, die eine Funktionalität des Feuchtesensors beeinträchtigen können.

Der gasdurchlässige Wandbereich kann sich über wenigstens einen Teil einer Oberfläche des Schutzgehäuses erstrecken. Insbesondere kann sich der gasdurchlässige Wandbereich über weniger als die Hälfte der Oberfläche, über mehr als die Hälfte der Oberfläche oder über die gesamte Oberfläche des Schutzgehäuses erstrecken. Der gasdurchlässige Wandbereich kann eine Metallsinterkomponente umfassen. Zusätzlich oder alternativ dazu kann der gasdurchlässige Wandbereich Edelstahl, insbesondere eine Edelstahlsinterkomponente, umfassen.

Die Kapselvorrichtung kann zur Verwendung in einem Messvolumen unter jedwedem Druck wenigstens im Bereich von 1 bar bis 100 bar (siehe Seite 14), insbesondere im Bereich von 0,5 bar bis 300 bar, insbesondere im Bereich von 0 bar bis 300 bar, ausgebildet sein.

Die Kapselvorrichtung kann wenigstens ein Befestigungselement zum Befestigen der Kapselvorrichtung an einer Durchführungsöffnung umfassen. Die Durchführungsöffnung kann in einer Wand eines Messvolumens zum Einführen des beheizbaren Sensors in das Messvolumen angeordnet sein. Das Befestigungselement kann wenigstens ein Gewinde umfassen. Das Messvolumen kann einen Kessel und/oder eine Leitung umfassen.

Alternativ dazu kann die Kapselvorrichtung wenigstens ein Befestigungselement zur freien Montage der Kapselvorrichtung in dem Messvolumen umfassen.

Die Kapselvorrichtung kann eine Wechselarmatur umfassen. Die Wechselarmatur kann ein Einführen des Sensors in den Aufnahmeraum der Kapselvorrichtung und/oder ein Entfernen des Sensors aus dem Aufnahmeraum der Kapselvorrichtung durch die Durchführungsöffnung gestatten. Ferner kann die Wechselarmatur ein reversibles Verschließen der Durchführungsöffnung bei entferntem Sensor gestatten. Das Befestigungselement der Kapselvorrichtung kann an der Wechselarmatur angeordnet sein.

Gemäß einem weiteren Aspekt wird ein Sensorsystem beschrieben. Das Sensorsystem umfasst einen Sensor und eine Kapselvorrichtung der hier vorgestellten Art. Dabei ist der Aufnahmeraum der Kapselvorrichtung zur Aufnahme des Sensors ausgebildet.

Der Sensor des Sensorsystems kann in dem Aufnahmeraum der Kapselvorrichtung angeordnet sein. Dabei kann der Sensor mittels eines Glasvergusses mit der Kapselvorrichtung verschmolzen sein. Der Glasverguss kann eine Dicke von 3 mm oder mehr aufweisen.

Der Sensor kann ein beheizbarer Sensor sein. Zudem kann die Kapselvorrichtung zum Verteilen und Abführen einer von dem beheizbaren Sensor abgegebenen Wärme ausgebildet sein. Das Verteilen und Abführen der Wärme kann dabei derart erfolgen, dass bei vorgesehenen Messbedingungen des Sensorsystems eine größte Oberflächentemperatur an einer Außenseite der Kapselvorrichtung geringer ist als eine größte Oberflächentemperatur des beheizbaren Sensors. Die vorgesehenen Messbedingungen können eine Umgebungstemperatur des Sensorsystems in einem vorgesehenen Temperaturbereich und/oder einen Umgebungsdruck des Sensorsystems in einem vorgesehenen Druckbereich umfassen.

Der beheizbare Sensor kann eine elektrische Leistung aufweisen, die größer ist als eine Grenzwertleistung für Eigensicherheit als Zündschutzart in Bezug auf den beheizbaren Sensor. Dabei kann die größte Oberflächentemperatur an der Außenseite der Kapselvorrichtung geringer sein als eine Grenzwerttemperatur für Explosionsschutz in Bezug auf den beheizbaren Sensor. Die Grenzwerttemperatur für Explosionsschutz kann dabei einer Grenzwerttemperatur für Eigensicherheit als Zündschutzart entsprechen. Zusätzlich oder alternativ dazu können dabei die Grenzwertleistung und/oder die Grenzwerttemperatur durch die technischen Normen EN60079-11 und EN60079-0 und/oder die technischen Normen IEC60079-11 und IEC60079-0 wenigstens teilweise, insbesondere vollständig, bestimmt sein.

Gemäß einem weiteren Aspekt wird ein Verfahren zum Betreiben eines beheizbaren Sensors, insbesondere in einer explosionsfähigen Atmosphäre, beschrieben. Das Verfahren umfasst Bereitstellen, in einem Messvolumen, einer Kapselvorrichtung für einen beheizbaren Sensor, wobei die Kapselvorrichtung ein Schutzgehäuse mit wenigstens einem gasdurchlässigen Wandbereich, der einen Gasaustausch zwischen einem Innenraum der Kapselvorrichtung und einer Umgebung der Kapselvorrichtung durch den gasdurchlässigen Wandbereich gestattet, und ein mit einem Füllgut befülltes Löschvolumen umfasst, das entlang einer Innenseite des Schutzgehäuses angeordnet ist und das einen zur Aufnahme des beheizbaren Sensors vorgesehenen Aufnahmeraum der Kapselvorrichtung wenigstens teilweise umgibt, und ein gasdurchlässiges Filterelement, das zwischen dem Löschvolumen und dem Aufnahmeraum angeordnet ist und das Löschvolumen in Bezug auf dem Aufnahmeraum begrenzt, und eines beheizbaren Sensors, der in dem Aufnahmeraum der Kapselvorrichtung angeordnet ist. Die Kapselvorrichtung ist zum Verteilen und Abführen einer von dem beheizbaren Sensor abgegebenen Wärme ausgebildet. Dabei kann die Kapselvorrichtung derart ausgebildet sein, dass bei vorgesehenen Betriebsbedingungen eine größte Oberflächentemperatur an einer Außenseite der Kapselvorrichtung niedriger ist als eine größte Oberflächentemperatur des beheizbaren Sensors. Das Verfahren umfasst ferner Beaufschlagen des beheizbaren Sensors mit elektrischer Leistung, wobei die elektrische Leistung derart bemessen ist, dass sie größer ist als eine Grenzwertleistung für Eigensicherheit als Zündschutzart in Bezug auf den beheizbaren Sensor. Die elektrische Leistung kann dabei ferner derart bemessen sein, dass die größte Oberflächentemperatur an der Außenseite der Kapselvorrichtung geringer ist als eine Grenzwerttemperatur für Explosionsschutz in Bezug auf den beheizbaren Sensor.

Die Grenzwerttemperatur für Explosionsschutz kann dabei einer Grenzwerttemperatur für Eigensicherheit als Zündschutzart entsprechen.

Weitere Einzelheiten und Vorteile der Erfindung werden bei nachfolgender Beschreibung von Ausführungsbeispielen anhand der Figuren deutlich werden.

Es zeigen:
- Fig. 1: eine Kapselvorrichtung für einen beheizbaren Sensor gemäß einem Ausführungsbeispiel;
- Fig. 2: ein Sensorsystem umfassend eine Kapselvorrichtung und einen beheizbaren Sensor gemäß einem Ausführungsbeispiel;
- Fig. 3: eine Kapselvorrichtung für einen beheizbaren Sensor gemäß einem weiteren Ausführungsbeispiel;
- Fig. 4A: ein Sensorsystem umfassend eine Kapselvorrichtung und einen beheizbaren Sensor gemäß einem weiteren Ausführungsbeispiel in einer ersten Position;
- Fig. 4B: das Sensorsystem aus Fig. 4A in einer zweiten Position;
- Fig. 5: ein Flussdiagramm für ein Verfahren zum Betreiben eines beheizbaren Sensors.

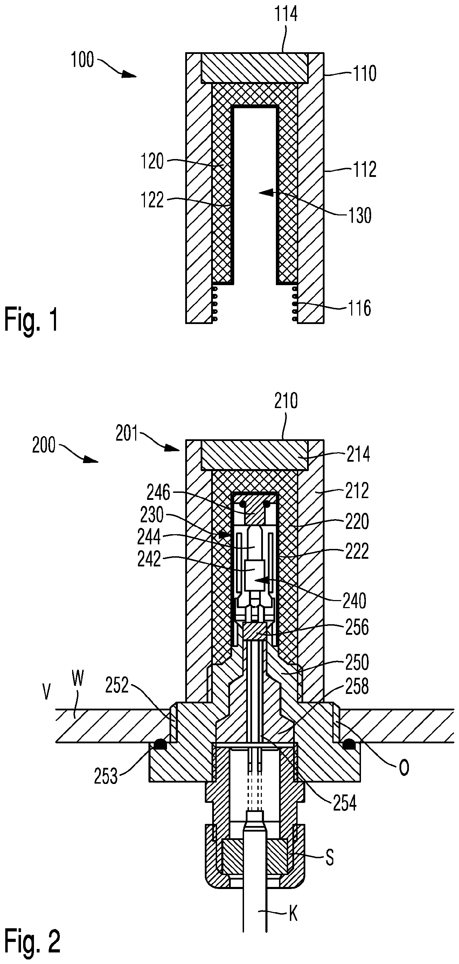

Fig. 1 zeigt eine schematische Darstellung einer Kapselvorrichtung 100. Die Kapselvorrichtung 100 umfasst ein Schutzgehäuse 110 mit einer Gehäusewand 112, in der ein gasdurchlässiger Wandbereich 114 angeordnet ist. An einer Innenseite des Schutzgehäuses 110 ist ein mit einem Füllgut befülltes Löschvolumen 120 angeordnet. Das Löschvolumen 120 ist mittels eines gasdurchlässigen Filterelements 122 in Bezug auf einen Aufnahmeraum 130 der Kapselvorrichtung 100 begrenzt. In dem gezeigten Beispiel umfasst die Kapselvorrichtung 100 außerdem ein Kapselgewinde 116 zur reversiblen Befestigung der Kapselvorrichtung 100, beispielsweise an einem Sensorgehäuse.

Die Kapselvorrichtung 100 ist zum Betreiben eines Sensors, insbesondere eines beheizbaren Sensors in explosionsgefährdeten Bereichen, vorgesehen. Dazu ist der Aufnahmeraum 130 zur Aufnahme eines Sensors ausgebildet. Insbesondere sind das Schutzgehäuse 110 und das Löschvolumen 120 so ausgebildet, dass sie eine Oberfläche des Sensors gegenüber einer Umgebung der Kapselvorrichtung 100 derart umgeben, dass eine Zündgefahr ausgehend von dem Sensor bezüglich eines explosionsfähigen Gasgemisches in der Umgebung der Kapselvorrichtung 100 verringert ist. Dabei erfüllt das Löschvolumen 120 wenigstens einige der eingangs beschriebenen Funktionen einer Sandkapselung. So ist durch das Löschvolumen 120 ein freies Volumen im Inneren der Kapselvorrichtung 100, in dem sich im Explosionsfall ein Druck aufbauen kann, verringert. Gleichzeitig bewirkt das in dem Löschvolumen 120 vorhandene Füllgut eine Abkühlung entzündeter Gase, bevor diese an die Gehäusewand 112 und gegebenenfalls durch den gasdurchlässigen Wandbereich 114 nach außen gelangen.

Bei einer herkömmlichen Sandkapselung ist die einzukapselnde Komponente üblicherweise in einem Gehäuse unmittelbar mit Füllgut verschüttet. Im Unterschied dazu bewirkt das Filterelement 122 der Kapselvorrichtung 100, dass das Löschvolumen 120 gegenüber dem Aufnahmeraum 130 und damit auch gegenüber einem darin gegebenenfalls angeordneten Sensor abgegrenzt ist. Dies ermöglicht eine separate Bereitstellung und Handhabung der Kapselvorrichtung 100 einschließlich des Löschvolumens 120. In Verbindung mit entsprechend ausgebildeten Sensoren bzw. Sensorgehäusen gestattet dies, dass ein Sensor je nach Bedarf und Einsatzbedingungen auf einfache Weise mit einem Löschvolumen als Kapselung versehen werden kann, etwa durch Aufschrauben der Kapselvorrichtung 100 auf ein Gewinde des Sensorgehäuses mittels des Kapselgewindes 116. Gleichzeitig ist durch Entfernen der Kapselvorrichtung 100 von dem Sensorgehäuse ein einfacher Zugriff auf den Sensor, etwa zu Austausch- oder Wartungszwecken, möglich, ohne dass dadurch das Löschvolumen beeinträchtigt oder beschädigt wird.

Das Filterelement 122 verhindert, dass Füllgut aus dem Löschvolumen 120 in den Aufnahmeraum 130 gelangt. Dadurch kann vermieden werden, dass Teile des Füllguts zumindest mit bestimmten Bereichen eines eingesetzten Sensors in Kontakt kommen. Dies gestattet eine Verwendung der Kapselvorrichtung 100 auch mit solchen Sensoren, die für ein direktes Verschütten mit Füllgut im Zusammenhang mit einer herkömmlichen Sandkapselung nicht geeignet sind. Dies ist beispielsweise bei einigen mechanisch empfindlichen Sensorelementen der Fall sowie bei Sensoren, die zu ihrem Betrieb ein freies Sensorvolumen benötigen, welches durch eine herkömmliche Sandkapselung versperrt würde. In einigen Beispielen ist dabei auch das Filterelement 122 so gestaltet, dass es mit mechanisch empfindlichen Bereichen des zu verwendenden Sensors nicht in Kontakt gerät.

Das Füllgut in dem Löschvolumen 120 umfasst in einigen Beispielen Sand und/oder Glasperlen. Eine kleinste Korngröße des Füllguts sowie eine größte Porengröße des Filterelements 122 sind dabei so aufeinander abgestimmt, dass keine Teile des Füllguts durch Poren des Filterelements 122 gelangen. Zudem ist das Filterelement 122 in einigen Beispielen hinreichend stark ausgebildet, dass eine vorbestimmte Position des Filterelements 122 gegenüber einem in dem Aufnahmeraum 130 angeordneten Sensor gewährleistet ist, beispielsweise ein vorbestimmter Abstand zwischen einem Sensorelement des Sensors und dem Filterelement 122. Das Filterelement 122 ist dabei beispielsweise als Drahtgitterfilter ausgebildet.

In weiteren Beispielen ist das Filterelement 122 hinreichend flexibel, um ein Anschmiegen des Löschvolumens 120 zumindest an Teile einer Oberfläche eines in der Kapselvorrichtung 100 aufgenommenen Sensors zu ermöglichen. So lässt sich ein freies Volumen an der Oberfläche des Sensors, in dem sich im Explosionsfall ein Druck aufbaut, reduzieren. Das Filterelement 122 umfasst dabei beispielsweise flexibles Gewebe, wie etwa flexibles Kunststoffgewebe.

In einigen Beispielen sind das Löschvolumen 120 und das Filterelement 122 hinsichtlich ihrer Materialien und Abmessungen so gestaltet, dass sie in Bezug auf den vorgesehenen Sensor technischen Anforderungen an eine Sandkapselung als Zündschutzart genügen. Beispiele derartiger Anforderungen finden sich in der europäischen Norm EN60079-5 und der entsprechenden internationalen Norm IEC60079-5.

Das Schutzgehäuse 110 begrenzt das Löschvolumen 120 in Bezug auf eine Umgebung der Kapselvorrichtung 100. Das Schutzgehäuse 110 besteht beispielsweise aus Metall, insbesondere Edelstahl, und/oder anderem mechanisch widerstandsfähigen Material. Der gasdurchlässige Wandbereich 114 des Schutzgehäuses 110 gestattet einen Gasaustausch zwischen einer Umgebung der Kapselvorrichtung 100 und einem darin angeordneten Sensor. Dabei erfolgt der Gasaustausch durch das Löschvolumen 120 einschließlich des darin vorhandenen Füllguts sowie durch das Filterelement 122. Der gasdurchlässige Wandbereich 114 umfasst in einigen Beispielen eine Metallsinterkomponente, beispielsweise eine gasdurchlässige Edelstahlsinterkomponente.

Zusätzlich zu dem Löschvolumen 120 verbessert auch das Schutzgehäuse 110 eine Zündschutzwirkung der Kapselvorrichtung 100. So dämmt das Schutzgehäuse 110 eine im Inneren der Kapselvorrichtung 100 eventuell erfolgende Zündung explosionsfähiger Gase gegenüber einer Umgebung der Kapselvorrichtung 100 wenigstens teilweise ein. Während der gasdurchlässige Wandbereich 114 dabei einen Gasaustausch zwischen einer Umgebung der Kapselvorrichtung 100 und der Sensoroberfläche ermöglicht, sind die Poren bzw. Kanäle innerhalb des gasdurchlässigen Wandbereich 114 in einigen Beispielen derart bemessen, dass im Fall einer Explosion innerhalb der Kapselvorrichtung 100 austretende Explosionsgase hinreichend abgekühlt werden, um eine Zündgefahr in der Umgebung der Kapselvorrichtung 100 durch die austretenden Gase zu verringern oder auszuschließen.

In dem gezeigten Beispiel erstreckt sich der gasdurchlässige Wandbereich 114 an einer Oberseite des Schutzgehäuses 110. In anderen Beispielen umfasst der gasdurchlässige Wandbereich 114 weitere und/oder andere Teile der Oberfläche des Schutzgehäuses 110. So erstreckt sich der gasdurchlässige Wandbereich 114 in einigen Beispielen zumindest im Wesentlichen über eine gesamte Oberfläche des Schutzgehäuses 110, insbesondere zumindest im Wesentlichen über einen gesamten Bereich des Löschvolumens 120.

In einigen Beispielen ist das Schutzgehäuse 110 derart ausgebildet, dass es den technischen Anforderungen an eine druckfeste Kapselung als Zündschutzart gemäß an dem vorgesehenen Einsatzort geltenden technischen Normen erfüllt. Beispiele derartiger Normen sind die europäische Norm EN60079-1 und die entsprechende internationale Norm die IEC60079-1.

In einigen Beispielen ist dabei das Löschvolumen 120 gleichzeitig so ausgebildet, dass es am vorgesehenen Einsatzort geltenden technischen Normen für eine Sandkapselung als Zündschutzart in Bezug auf den vorgesehenen Sensor entspricht, wie vorangehend beschrieben. Bei diesen Beispielen der Kapselvorrichtung 100 sind so wenigstens zwei Zündschutzarten in Kombination verwirklicht. Dies gestattet eine Verwendung der Kapselvorrichtung 100 beispielsweise mit beheizbaren Sensoren unter Bedingungen, bei denen eine Redundanz von Zündschutzarten vorteilhaft und/oder erforderlich ist, etwa hinsichtlich eines Zweifehlerfalls. Die Kapselvorrichtung 100 gestattet auf diese Weise eine Ausweitung der Anwendungsmöglichkeiten bestimmter Sensoren in explosionsgefährdeten Bereichen, beispielsweise in Zonen mit erhöhten Sicherheitsanforderungen durch häufigeres Vorliegen explosionsfähiger Atmosphären. Vorschriften bezüglich einer Kombination mehrerer Zündschutzarten sind beispielsweise in der europäischen technischen Norm EN60079-26 sowie der entsprechenden internationalen Norm IEC60079-26 definiert.

Fig. 2 zeigt eine schematische Darstellung eines Sensorsystems 200 mit einer Kapselvorrichtung 201 und einem darin angeordneten Sensor 240. Bei der Kapselvorrichtung 201 handelt es sich beispielsweise um eine Kapselvorrichtung wie im Zusammenhang mit Fig. 1 beschrieben. Zudem ist die Kapselvorrichtung 201 zur Aufnahme des Sensors 240 ausgebildet.

Die Kapselvorrichtung 201 des Sensorsystems 200 umfasst ein Schutzgehäuse 210 mit einer Gehäusewand 212 und einem darin angeordneten gasdurchlässigen Wandbereich 214. Zudem ist entlang einer Innenseite des Schutzgehäuses 210 ein Löschvolumen 220 angeordnet, das mittels eines Filterelements 222 gegenüber einem Aufnahmeraum 230 der Kapselvorrichtung 201 abgegrenzt ist. Bezüglich dieser Merkmale gilt das im Zusammenhang mit der Kapselvorrichtung 100 aus Fig. 1 Gesagte entsprechend.

Der Sensor 240 ist in dem Aufnahmeraum 230 der Kapselvorrichtung 201 angeordnet und umfasst in dem gezeigten Beispiel ein Sensorelement 242 angrenzend an ein Sensorvolumen 244, das durch eine Filterkappe 246 begrenzt ist. Das Sensorelement 242 ist auf einem Gehäusekörper 250 angeordnet und mittels Metallstiften 254, die sich durch den Gehäusekörper 250 erstrecken, mit einem Kabel K elektrisch verbunden.

Das Schutzgehäuse 210 ist mit dem Gehäusekörper 250 mechanisch widerstandsfähig verbunden, beispielsweise mittels eines oder mehrerer Gewinde. In dem gezeigten Beispiel ist das Sensorsystem 200 außerdem für Messungen in einem Messvolumen V angeordnet. Dabei ist das Schutzgehäuse 210 durch eine Durchführungsöffnung O in einer Wand W des Messvolumens V geführt. Zudem ist der Gehäusekörper 250 mit der Wand W des Messvolumens V mittels wenigstens eines Befestigungselements 252, beispielsweise mittels eines oder mehrerer Gewinde, mechanisch widerstandsfähig verbunden. In dem gezeigten Beispiel umfasst das Sensorsystem 200 ferner eine Wanddichtung 253, die dazu vorgesehen ist, einen Übergang zwischen der Wand W und dem Gehäusekörper 250 abzudichten.

Der Sensor 240 ist in dem gezeigten Beispiel mit dem Gehäusekörper 250 der Kapselvorrichtung 201 mittels eines Glasvergusses 256 irreversibel verschmolzen. Dabei sind die Metallstifte 254 zur elektrischen Kontaktierung des Sensors 240 durch den Glasverguss 256 geführt. In einigen Beispiele ist der Glasverguss 256 gemäß geltenden technischen Anforderungen, beispielsweise Anforderungen an eine druckfeste Kapselung als Zündschutzart, ausgebildet. Insbesondere weist der Glasverguss 256 in einigen Beispielen eine Dicke von wenigstens 3 mm auf.

In einigen Beispielen ist das Sensorsystem 200, insbesondere die Kapselvorrichtung 201, zur Verwendung in Messvolumina V über große Druckbereiche ausgebildet. Der Druckbereich umfasst dabei in einigen Beispielen jedweden Druck zwischen 1 bar und 100 bar, beispielsweise jedweden Druck zwischen 0,5 bar und 300 bar, insbesondere jedweden Druck zwischen 0 bar und 300 bar.

Die Metallstifte 254 sind außerdem mittels eines Vergusses 258 in dem Gehäusekörper 250 befestigt. Der Verguss 258 umfasst dabei in einigen Beispiel ein vom Material des Glasvergusses 256 verschiedenes Material, beispielsweise Kunststoff und/oder Harz. Zum Zweck einer Zugentlastung einer Verbindung der Metallstifte 254 mit dem Kabel K ist in dem gezeigten Beispiel zudem eine Kabelverschraubung S vorgesehen.

Bei dem Sensor 240 handelt es sich in einigen Beispielen um einen nach am vorgesehenen Einsatzort geltenden technischen Normen eigensicheren Sensor, d.h. eine Leistungsaufnahme des Sensors 240 ist derart begrenzt, dass sich keine zündgefährlich heißen Oberflächen oder zündgefährliche elektrische Entladungen an dem Sensor 240 ergeben können. Bei geeigneter Ausbildung des Löschvolumens 220 hinsichtlich einer Sandkapselung und/oder des Schutzgehäuses 210 hinsichtlich einer druckfesten Kapselung gestattet das Sensorsystem 200 in diesen Fällen zusätzliche Kombinationen, und damit Redundanzen, verwirklichter Zündschutzarten. Beispielsweise lassen sich so wenigstens drei Zündschutzarten, nämlich Eigensicherheit, Sandkapselung und druckfeste Kapselung, gleichzeitig verwirklichen. Das Sensorsystem 200 gestattet so eine weitere Ausweitung der Anwendungsmöglichkeiten verschiedener Sensoren 240 in Einsatzgebieten, in denen eine entsprechende Kombination von Zündschutzarten vorteilhaft und/oder durch technische Bedingungen oder Vorschriften erforderlich ist.

Das Schutzgehäuse 210 ist in einigen Beispielen so beschaffen, dass es eine von einem beheizbaren Sensor 240 bei dessen Betrieb abgegebene Wärme aufnimmt und an das Messvolumen V bzw. an eine Umgebung des Messvolumens V abführt. Infolge von Wärmeverteilung und Wärmeabgabe über die Oberfläche des Schutzgehäuses 210 an die Umgebung ist eine größte Oberflächentemperatur an einer Außenseite des Schutzgehäuses 210 geringer als eine größte Oberflächentemperatur des Sensors 240. Das Schutzgehäuse 210 gestattet auf diese Weise das sichere Betreiben beheizbarer Sensoren 240 in explosionsgefährdeten Bereichen auch bei solchen Oberflächentemperaturen des Sensors 240, die einen Betrieb des Sensors 240 allein sonst nicht sicher und/oder zulässig machen würden. Insbesondere sind in einigen Beispielen des Sensorsystems 200 die Kapselvorrichtung 201 so gestaltet und eine Leistungsaufnahme des beheizbaren Sensors 240 so gewählt, dass unter vorgesehenen Messbedingungen in dem Messvolumen V, beispielsweise hinsichtlich einer Umgebungstemperatur und eines Umgebungsdrucks in dem Messvolumen V, die Leistungsaufnahme des Sensors 240 höher ist, als es den Anforderungen an Eigensicherheit als Zündschutzart in Bezug auf den Sensor 240 entsprechen würde, wohingegen eine größte Oberflächentemperatur an einer Außenseite der Kapselvorrichtung 201 weniger beträgt als eine Grenzwerttemperatur für Explosionsschutz. Der beheizbare Sensor 240 umfasst dabei beispielsweise ein Taupunktmessgerät mit Feuchtesensor, der eine Ausheizfunktion aufweist. Auf die vorangehend beschriebene Weise begünstigt das Sensorsystem 200 die Verwendung beheizbarer Sensoren 240 unter Einsatzbedingungen, unter denen dies anderenfalls technischen Anforderungen und/oder Vorschriften nicht entsprechen würde.

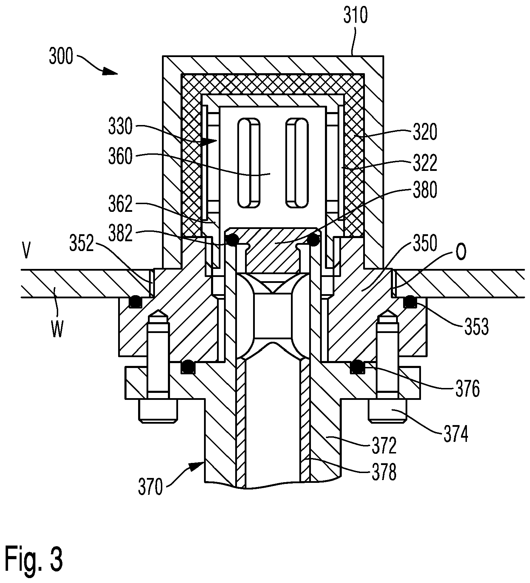

Fig. 3 zeigt eine schematische Darstellung einer weiteren Kapselvorrichtung 300. Ähnlich den vorgenannten Beispielen umfasst die Kapselvorrichtung 301 ein Schutzgehäuse 310, das wenigstens einen gasdurchlässigen Wandbereich umfasst, und ein entlang einer Innenseite des Schutzgehäuses 310 angeordnetes Löschvolumen 320, das durch ein Filterelement 322 gegenüber einem Aufnahmeraum 330 der Kapselvorrichtung 300 begrenzt ist. Das Schutzgehäuse 310 ist zudem mit einem Gehäusekörper 350 verbunden. Der Gehäusekörper 350 ist durch ein Befestigungsmittel 352 in einer Durchführungsöffnung O der Wand W eines Messvolumens V befestigt und gegenüber der Wand W mit einer Wanddichtung 353 abgedichtet. Soweit sich aus dem Nachfolgenden nichts anderes ergibt, gilt bezüglich dieser Merkmale das im Zusammenhang mit den vorangehenden Beispielen aus den Fign. 1 und 2 Gesagte entsprechend.

Abweichend von dem Sensorsystem 200 aus Fig. 2 ist die Kapselvorrichtung 300 für den Ein- und Ausbau eines Sensors bei montierter Kapselvorrichtung 300 ausgebildet. Zu diesem Zweck umfasst die Kapselvorrichtung 300 eine Wechselarmatur 370, die an einer Außenseite des Gehäusekörpers 350 mit Schrauben 374 befestigt und einer Armaturdichtung 376 abgedichtet ist. Die Wechselarmatur 370 weist ein Schubrohr 378 auf. Das Schubrohr 378 erstreckt sich durch den Armaturkörper 372 und den Gehäusekörper 350 bis zu einem Einbauvolumen 360, das sich innerhalb des Aufnahmeraums 330 der Kapselvorrichtung 300 befindet. Das Schubrohr 378 ist dazu vorgesehen, bei montierter Kapselvorrichtung 300 von einer Außenseite des Messvolumens einen Sensor durch das Schubrohr 378 in das Einbauvolumen 360 zu führen bzw. aus diesem zu entfernen. Bei der gezeigten Wechselarmatur 370 handelt es sich beispielsweise um eine Wechselarmatur der Firma POSTBERG + Co. GmbH, wie in der EP 1148317B1 beschrieben.

Die Kapselvorrichtung 300 weist am inneren Ende des Schubrohrs 378 eine Schubrohrendkapsel 380 auf, die mittels einer Schubrohrdichtung 382 gegenüber dem Armaturkörper 372 abgedichtet ist. Die Schubrohrendkapsel 380 gestattet bei entferntem Sensor, wie in Fig. 3 dargestellt, ein druckfestes Verschließen des Aufnahmeraums 330 gegenüber einer Umgebung des Messvolumens V. Bei Einsetzen eines Sensors durch das Schubrohr 378 wird dagegen die Schubrohrendkapsel 380 gleichzeitig in eine geöffnete Position verschoben.

Die Kapselvorrichtung 300 begünstigt gegenüber den zuvor beschriebenen Beispielen ein Einsetzen, Entfernen und/oder Austauschen eines Sensors von außerhalb des Messvolumens V. Beispielsweise gestattet die Kapselvorrichtung 300 eine einfache Wartung des verwendeten Sensors, ein Ersetzen bei Defekt oder ein Austauschen durch einen anderen Sensor, etwa bei geänderten Messanforderungen.

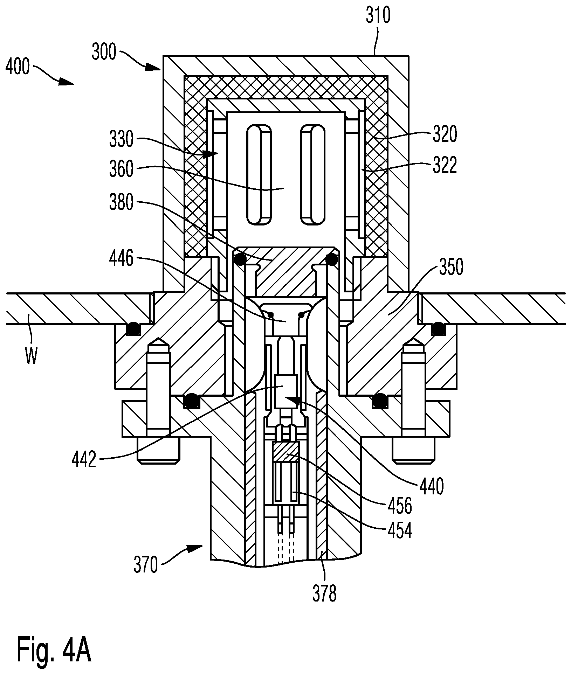

Fig. 4A zeigt ein weiteres Beispiel eines Sensorsystems 400. Das Sensorsystem 400 umfasst eine Kapselvorrichtung 300 und einen Sensor 440. In dem gezeigten Beispiel handelt es sich bei der Kapselvorrichtung 300 um eine Kapselvorrichtung wie im Zusammenhang mit Fig. 3 beschrieben. Gleiche Bezugszeichen wie in Fig. 3 bezeichnen dabei gleiche Merkmale.

Der Sensor 440 ist in dem Schubrohr 378 der Kapselvorrichtung 300 angeordnet. Der Sensor 440 entspricht beispielsweise dem Sensor 240, wie im Zusammenhang mit Fig. 2 beschrieben. Insbesondere weist auch der Sensor 440 ein Sensorelement 442 auf sowie eine Filterkappe 446 auf, die ein Sensorvolumen begrenzt. Ferner sind Metallstifte 454 zur elektrischen Kontaktierung des Sensorelements 442 vorgesehen, die mittels eines Vergusses, beispielsweise eines Glasvergusses 456, eingefasst sind.

Bei der in Fig. 4A gezeigten Position des Sensors 440 ist dieser aus dem Aufnahmeraum 330 des Sensorsystems 400 entfernt. Dabei verschließt die Schubrohrendkapsel 380 den Aufnahmeraum 330 gegenüber dem Sensor 440. In der gezeigten Position befindet sich das Sensorsystem 400 in nicht-messbereitem Zustand. Eine solche Position ergibt sich beispielsweise während eines Einsetzens oder Entfernens des Sensors aus dem Messvolumen V.

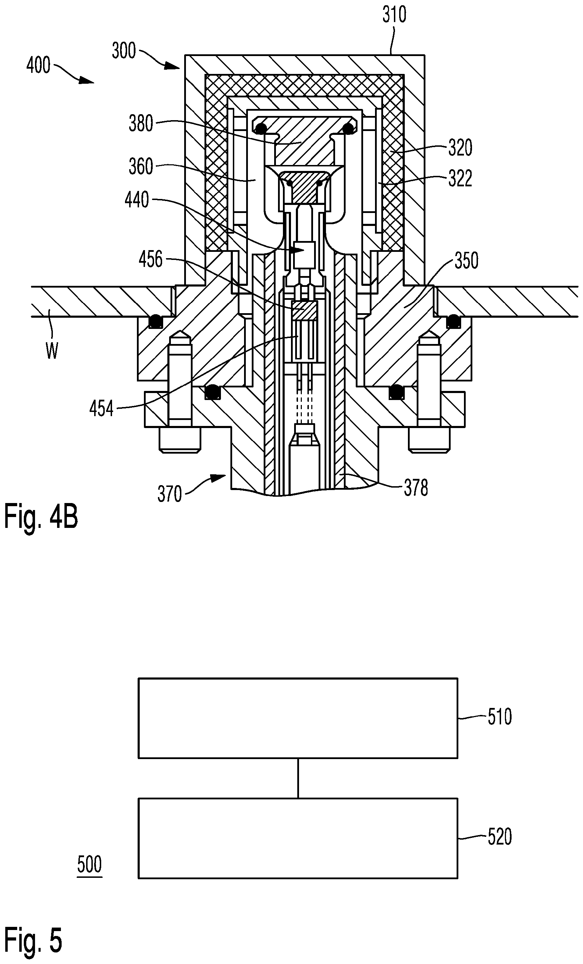

Fig. 4B zeigt eine weitere schematische Ansicht des Sensorsystems 400. Abweichend von Fig. 4A ist das Sensorsystem 400 dabei in einer zweiten Position gezeigt. In dieser Position ist der Sensor 440 in den Aufnahmeraum 330 der Kapselvorrichtung 300 eingeschoben. Gleichzeitig ist dabei die Schubrohrendkapsel 380 ebenfalls in eine geöffnete Position verschoben. In der gezeigten Position befindet sich das Sensorsystem 400 in messbereitem Zustand. In der gezeigten Position ist so ein Gasaustausch zwischen einer Umgebung des Schutzgehäuses 310 und dem Sensorvolumen des Sensors 440 ermöglicht.

Einige der vorangehend dargestellten Techniken sind im Zusammenhang mit beheizbaren Sensoren beschrieben. Es versteht sich jedoch, dass die beschriebenen Techniken auch im Zusammenhang mit nicht beheizbaren Sensoren und/oder anderen funktionalen Komponenten zumindest einige der genannten Vorteile bieten.

Fig. 5 zeigt ein Flussdiagramm für ein Verfahren 500 zum Betreiben eines beheizbaren Sensors, insbesondere in einer explosionsfähigen Atmosphäre. Das Verfahren 500 umfasst ein Bereitstellen einer Kapselvorrichtung für einen beheizbaren Sensor in einem Messvolumen sowie eines beheizbaren Sensors in einem Aufnahmeraum der Kapselvorrichtung, Schritt 510. Die Kapselvorrichtung umfasst dabei ein Schutzgehäuse mit einem gasdurchlässigen Wandbereich, der einen Gasaustausch zwischen einem Aufnahmeraum der Kapselvorrichtung und einer Umgebung der Kapselvorrichtung durch den gasdurchlässigen Wandbereich gestattet, sowie ein mit einem Füllgut befülltes Löschvolumen, das entlang einer Innenseite des Schutzgehäuses angeordnet ist und das den zur Aufnahme des beheizbaren Sensors vorgesehenen Aufnahmeraum umgibt. Die Kapselvorrichtung ist dabei zum Verteilen und Abführen einer von dem beheizbaren Sensor abgegebenen Wärme ausgebildet, derart dass bei vorgesehenen Betriebsbedingungen eine größte Oberflächentemperatur an einer Außenseite der Kapselvorrichtung niedriger ist als eine größte Oberflächentemperatur des beheizbaren Sensors.

Das Verfahren 500 umfasst ferner ein Beaufschlagen des beheizbaren Sensors mit elektrischer Leistung, Schritt 520. Die elektrische Leistung ist dabei derart bemessen, dass sie größer ist als eine Grenzwertleistung für Eigensicherheit als Zündschutzart in Bezug auf den beheizbaren Sensor. Gleichzeitig ist die elektrische Leistung derart bemessen, dass die größte Oberflächentemperatur an der Außenseite der Kapselvorrichtung geringer ist als eine Grenzwerttemperatur für Explosionsschutz in Bezug auf den beheizbaren Sensor. Eine Temperaturdifferenz zwischen einer Oberfläche des Sensors und der Oberfläche der Kapselvorrichtung ergibt sich beispielsweise infolge von Wärmeverteilung und Wärmeabgabe über die Oberfläche der Kapselvorrichtung.

Bei der Kapselvorrichtung und dem beheizbaren Sensor handelt es sich beispielsweise um solche der vorangehenden beschriebenen Arten. Wie im Zusammenhang mit Fig. 2 beschrieben, gestattet das Verfahren 500 einen Einsatz beheizbarer Sensoren auch unter solchen Einsatzbedingungen, bei denen ein Betrieb eines solchen Sensors ohne Bereitstellung einer entsprechenden Kapselvorrichtung nicht hinreichend sicher und/oder zulässig wäre. Dies ist ermöglicht durch die gegenüber der Sensortemperatur geringere Oberflächentemperatur der Kapselvorrichtung.

## Patentansprüche

1. Kapselvorrichtung (100; 201; 300) für einen Sensor (240; 440), insbesondere zum Betreiben eines Sensors (240; 440) in einer explosionsfähigen Atmosphäre, umfassend:
- ein Schutzgehäuse (110; 210; 310) mit wenigstens einem gasdurchlässigen Wandbereich (114), der einen Gasaustausch zwischen einem Innenraum der Kapselvorrichtung (100; 201; 300) und einer Umgebung der Kapselvorrichtung durch den gasdurchlässigen Wandbereich (114) gestattet,
- ein mit einem Füllgut befülltes Löschvolumen (120; 220; 320), das entlang einer Innenseite des Schutzgehäuses (110; 210; 310) angeordnet ist und das einen zur Aufnahme eines Sensors (240; 440) vorgesehenen Aufnahmeraum (130; 230; 330) der Kapselvorrichtung (100; 201; 300) wenigstens teilweise umgibt, und
- ein gasdurchlässiges Filterelement (122; 222; 322), das zwischen dem Löschvolumen (120; 220; 320) und dem Aufnahmeraum (130; 230; 330) angeordnet ist und das Löschvolumen (120; 220; 320) in Bezug auf den Aufnahmeraum (130; 230; 330) begrenzt.

2. Kapselvorrichtung (100; 201; 300) nach Anspruch 1, wobei das Filterelement (122; 222; 322) ferner dazu ausgebildet ist, ein Eindringen von Füllgut aus dem Löschvolumen (120; 220; 320) in den Aufnahmeraum (130; 230; 330) zu verhindern.

3. Kapselvorrichtung (100; 201; 300) nach Anspruch 1 oder 2, wobei das Filterelement (122; 222; 322) ferner dazu ausgebildet ist, einen mechanischen Kontakt zwischen dem Füllgut und/oder dem Filterelement (122; 222; 322) und wenigstens einem Teil eines Sensors (240; 440) zu verhindern, wenn der Sensor (240; 440) in dem Aufnahmeraum (130; 230; 330) angeordnet ist.

4. Kapselvorrichtung (100; 201; 300) nach einem der vorhergehenden Ansprüche, wobei das Löschvolumen (120; 220; 320) so beschaffen ist, dass es in Bezug auf den Sensor (240; 440) den Anforderungen an eine Sandkapselung als eine erste Zündschutzart entspricht, und dass das Schutzgehäuse (110; 210; 310) so beschaffen ist, dass es in Bezug auf den Sensor (240; 440) den Anforderungen an eine druckfeste Kapselung als eine weitere Zündschutzart entspricht.

5. Kapselvorrichtung (100; 201; 300) nach einem der vorhergehenden Ansprüche, wobei der gasdurchlässige Wandbereich (114) eine Metallsinterkomponente umfasst.

6. Kapselvorrichtung (100; 201; 300) nach einem der vorhergehenden Ansprüche, wobei der Sensor (240; 440) ein beheizbarer Sensor ist.

7. Kapselvorrichtung (100; 201; 300) nach einem der vorhergehenden Ansprüche, wobei das Filterelement (122; 222; 322) einen Drahtgitterfilter umfasst, und/oder wobei das Füllgut Sand und/oder Glasperlen umfasst.

8. Kapselvorrichtung (100; 201; 300) nach einem der vorhergehenden Ansprüche, wobei die Kapselvorrichtung zur Verwendung in einem Messvolumen (V) unter jedwedem Druck im Bereich von 0 bar bis 300 bar ausgebildet ist.

9. Kapselvorrichtung (201; 300) nach einem der vorhergehenden Ansprüche, ferner umfassend wenigstens ein Befestigungselement (252; 352) zum Befestigen der Kapselvorrichtung (201; 300) an einer Durchführungsöffnung (O), die in einer Wand (W) eines Messvolumens (V) zum Einführen des Sensors (240; 440) in das Messvolumen (V) angeordnet ist.

10. Kapselvorrichtung (300) nach Anspruch 9, ferner umfassend eine Wechselarmatur (360), die ein Einführen des Sensors (440) in den Aufnahmeraum (330) der Kapselvorrichtung (300) und/oder ein Entfernen des Sensors (440) aus dem Aufnahmeraum (330) der Kapselvorrichtung (300) durch die Durchführungsöffnung (O) sowie ein reversibles Verschließen der Durchführungsöffnung (O) bei entferntem Sensor (440) gestattet.

11. Sensorsystem (200; 400) umfassend einen Sensor (240; 440) und eine Kapselvorrichtung (201; 300) nach einem der vorhergehenden Ansprüche, wobei der Aufnahmeraum (230; 330) der Kapselvorrichtung zur Aufnahme des Sensors (240; 440) ausgebildet ist.

12. Sensorsystem (200) nach Anspruch 11, umfassend eine Kapselvorrichtung (201) nach einem der Ansprüche 1 bis 9, wobei der Sensor (240) in dem Aufnahmeraum (230) der Kapselvorrichtung (200) angeordnet und mittels eines Glasvergusses (256) mit der Kapselvorrichtung (201) verschmolzen ist.

13. Sensorsystem (200; 400) nach Anspruch 11 oder 12, wobei der Sensor (240; 440) ein beheizbarer Sensor ist und die Kapselvorrichtung (201; 300) zum Verteilen und Abführen einer von dem beheizbaren Sensor (240; 440) abgegebenen Wärme ausgebildet ist.

14. Sensorsystem (200; 400) nach Anspruch 13, wobei der beheizbare Sensor (240; 440) eine elektrische Leistung aufweist, die größer ist als eine Grenzwertleistung für Eigensicherheit als Zündschutzart in Bezug auf den beheizbaren Sensor (240; 440), und die größte Oberflächentemperatur an der Außenseite der Kapselvorrichtung (201; 300) geringer ist als eine Grenzwerttemperatur für Explosionsschutz in Bezug auf den beheizbaren Sensor (240; 440).

15. Verfahren (500) zum Betreiben eines beheizbaren Sensors, insbesondere in einer explosionsfähigen Atmosphäre, umfassend:
- Bereitstellen (510), in einem Messvolumen, einer Kapselvorrichtung für einen beheizbaren Sensor nach einem der Ansprüche 1 bis 9 und eines beheizbaren Sensors, der in dem Aufnahmeraum der Kapselvorrichtung angeordnet ist, wobei die Kapselvorrichtung zum Verteilen und Abführen einer von dem beheizbaren Sensor abgegebenen Wärme ausgebildet ist, und
- Beaufschlagen (520) des beheizbaren Sensors mit elektrischer Leistung, wobei die elektrische Leistung derart bemessen ist, dass sie größer ist als eine Grenzwertleistung für Eigensicherheit als Zündschutzart in Bezug auf den beheizbaren Sensor.

## Claims

1. Encapsulation device (100; 201; 300) for a sensor (240; 440), in particular for operating a sensor (240; 440) in an explosive atmosphere, comprising:
- a protective housing (110; 210; 310) having at least one gas-permeable wall region (114) that allows gas exchange between an interior of the encapsulation device (100; 201; 300) and an environment of the encapsulation device through the gas-permeable wall region (114),
- an extinguishing volume (120; 220; 320) filled with a filling material, said extinguishing volume being arranged along an inner side of the protective housing (110; 210; 310) and at least partly surrounding a receiving space (130; 230; 330) of the encapsulation device (100; 201; 300), said receiving space being provided for receiving a sensor (240; 440), and
- a gas-permeable filter element (122; 222; 322), which is arranged between the extinguishing volume (120; 220; 320) and the receiving space (130; 230; 330) and delimits the extinguishing volume (120; 220; 320) with respect to the receiving space (130; 230; 330).

2. Encapsulation device (100; 201; 300) according to Claim 1, wherein the filter element (122; 222; 322) is furthermore configured to prevent filling material from the extinguishing volume (120; 220; 320) from penetrating into the receiving space (130; 230; 330) .

3. Encapsulation device (100; 201; 300) according to Claim 1 or 2, wherein the filter element (122; 222; 322) is furthermore configured to prevent a mechanical contact between the filling material and/or the filter element (122; 222; 322) and at least one part of a sensor (240; 440) when the sensor (240; 440) is arranged in the receiving space (130; 230; 330) .

4. Encapsulation device (100; 201; 300) according to any of the preceding claims, wherein the extinguishing volume (120; 220; 320) is constituted such that it complies with the requirements regarding a powder filling as a first type of ignition protection with respect to the sensor (240; 440), and wherein the protective housing (110; 210; 310) is constituted such that it complies with the requirements regarding a flameproof enclosure as a further type of ignition protection with respect to the sensor (240; 440).

5. Encapsulation device (100; 201; 300) according to any of the preceding claims, wherein the gas-permeable wall region (114) comprises a sintered metal component.

6. Encapsulation device (100; 201; 300) according to any of the preceding claims, wherein the sensor (240; 440) is a heatable sensor.

7. Encapsulation device (100; 201; 300) according to any of the preceding claims, wherein the filter element (122; 222; 322) comprises a wire mesh filter, and/or wherein the filling material comprises sand and/or glass beads.

8. Encapsulation device (100; 201; 300) according to any of the preceding claims, wherein the encapsulation device is configured for use in a measurement volume (V) at any pressure in the range of from 0 bar to 300 bar.

9. Encapsulation device (201; 300) according to any of the preceding claims, furthermore comprising at least one securing element (252; 352) for securing the encapsulation device (201; 300) at a lead-through opening (O) arranged in a wall (W) of a measurement volume (V) for inserting the sensor (240; 440) into the measurement volume (V).

10. Encapsulation device (300) according to Claim 9, furthermore comprising an interchangeable fitting (360) allowing the sensor (440) to be inserted into the receiving space (330) of the encapsulation device (300) and/or allowing the sensor (440) to be removed from the receiving space (330) of the encapsulation device (300) through the lead-through opening (O) and also allowing the lead-through opening (O) to be reversibly closed when the sensor (440) is removed.

11. Sensor system (200; 400) comprising a sensor (240; 440) and an encapsulation device (201; 300) according to any of the preceding claims, wherein the receiving space (230; 330) of the encapsulation device is configured for receiving the sensor (240; 440) .

12. Sensor system (200) according to Claim 11, comprising an encapsulation device (201) according to any of Claims 1 to 9, wherein the sensor (240) is arranged in the receiving space (230) of the encapsulation device (200) and is fused with the encapsulation device (201) by means of a glass potting (256).

13. Sensor system (200; 400) according to Claim 11 or 12, wherein the sensor (240; 440) is a heatable sensor and the encapsulation device (201; 300) is configured for distributing and dissipating heat emitted by the heatable sensor (240; 440).

14. Sensor system (200; 400) according to Claim 13, wherein the heatable sensor (240; 440) has an electrical power greater than a limit power for intrinsic safety as the type of ignition protection with respect to the heatable sensor (240; 440), and the highest surface temperature at the outer side of the encapsulation device (201; 300) is lower than a limit temperature for explosion protection with respect to the heatable sensor (240; 440).

15. Method (500) for operating a heatable sensor, in particular in an explosive atmosphere, comprising:
- providing (510), in a measurement volume, an encapsulation device for a heatable sensor according to any of Claims 1 to 9 and a heatable sensor arranged in the receiving space of the encapsulation device, wherein the encapsulation device is configured for distributing and dissipating heat emitted by the heatable sensor, and
- applying (520) electrical power to the heatable sensor, wherein the electrical heatable sensor is dimensioned such that it is greater than a limit power for intrinsic safety as the type of ignition protection with respect to the heatable sensor.

## Revendications

1. Dispositif d'encapsulation (100 ; 201 ; 300) pour un capteur (240 ; 440), notamment destiné à faire fonctionner un capteur (240 ; 440) dans une atmosphère explosible, comprenant :
- un boîtier de protection (110 ; 210 ; 310) ayant au moins une zone de paroi (114) perméable au gaz, laquelle permet un échange de gaz entre un espace intérieur du dispositif d'encapsulation (100 ; 201 ; 300) et un environnement du dispositif d'encapsulation à travers la zone de paroi (114) perméable au gaz,
- un volume d'extinction (120 ; 220 ; 320) rempli d'une matière de remplissage, lequel est disposé le long d'un côté intérieur du boîtier de protection (110 ; 210 ; 310) et entoure au moins partiellement un espace d'accueil (130 ; 230 ; 330) du dispositif d'encapsulation (100 ; 201 ; 300) conçu pour accueillir un capteur (240 ; 440), et
- un élément filtrant (122 ; 222 ; 322) perméable au gaz, qui est disposé entre le volume d'extinction (120 ; 220 ; 320) et l'espace d'accueil (130 ; 230 ; 330) et qui délimite le volume d'extinction (120 ; 220 ; 320) par rapport à l'espace d'accueil (130 ; 230 ; 330).

2. Dispositif d'encapsulation (100 ; 201 ; 300) selon la revendication 1, l'élément filtrant (122 ; 222 ; 322) étant en outre configuré pour empêcher une pénétration de matière de remplissage du volume d'extinction (120 ; 220 ; 320) dans l'espace d'accueil (130 ; 230 ; 330).

3. Dispositif d'encapsulation (100 ; 201 ; 300) selon la revendication 1 ou 2, l'élément filtrant (122 ; 222 ; 322) étant en outre configuré pour empêcher un contact mécanique entre la matière de remplissage et/ou l'élément filtrant (122 ; 222 ; 322) et au moins une partie du capteur (240 ; 440) lorsque le capteur (240 ; 440) est disposé dans l'espace d'accueil (130 ; 230 ; 330).

4. Dispositif d'encapsulation (100 ; 201 ; 300) selon l'une des revendications précédentes, le volume d'extinction (120 ; 220 ; 320) étant fourni de telle sorte qu'il correspond, en référence au capteur (240 ; 440), aux exigences de remplissage pulvérulent en tant qu'un premier mode de protection « e », et en ce que le boîtier de protection (110 ; 210 ; 310) est fourni de telle sorte qu'il correspond, en référence au capteur (240 ; 440), aux exigences d'enveloppe antidéflagrante en tant qu'un mode de protection « e ».

5. Dispositif d'encapsulation (100 ; 201 ; 300) selon l'une des revendications précédentes, la zone de paroi (114) perméable au gaz comprenant un composant fritté métallique.

6. Dispositif d'encapsulation (100 ; 201 ; 300) selon l'une des revendications précédentes, le capteur (240 ; 440) étant un capteur chauffable.

7. Dispositif d'encapsulation (100 ; 201 ; 300) selon l'une des revendications précédentes, l'élément filtrant (122 ; 222 ; 322) comprenant un filtre en treillis métallique et/ou la matière de remplissage comprenant du sable et/ou des perles de verre.

8. Dispositif d'encapsulation (100 ; 201 ; 300) selon l'une des revendications précédentes, le dispositif d'encapsulation étant configuré pour une utilisation dans un volume de mesure (V) sous n'importe quelle pression dans la plage de 0 bar à 300 bars.

9. Dispositif d'encapsulation (201 ; 300) selon l'une des revendications précédentes, comprenant en outre au moins un élément de fixation (252 ; 352) destiné à fixer le dispositif d'encapsulation (201 ; 300) au niveau d'une ouverture de passage (O) qui est disposée dans une paroi (W) d'un volume de mesure (V) servant à l'introduction du capteur (240 ; 440) dans le volume de mesure (V).

10. Dispositif d'encapsulation (300) selon la revendication 9, comprenant un élément de robinetterie d'inversion (360) qui permet une introduction du capteur (440) dans l'espace d'accueil (330) du dispositif d'encapsulation (300) et/ou un retrait du capteur (440) hors de l'espace d'accueil (330) du dispositif d'encapsulation (300) à travers l'ouverture de passage (O) ainsi qu'une fermeture réversible de l'ouverture de passage (O) lorsque le capteur (440) est retiré.

11. Système capteur (200 ; 400) comprenant un capteur (240 ; 440) et un dispositif d'encapsulation (201 ; 300) selon l'une des revendications précédentes, l'espace d'accueil (230 ; 330) du dispositif d'encapsulation étant configuré pour accueillir le capteur (240 ; 440).

12. Système capteur (200) selon la revendication 11, comprenant un dispositif d'encapsulation (201) selon l'une des revendications 1 à 9, le capteur (240) étant disposé dans l'espace d'accueil (230) du dispositif d'encapsulation (200) et étant fusionné avec le dispositif d'encapsulation (201) au moyen d'un enrobage en verre.

13. Système capteur (200 ; 400) selon la revendication 11 ou 12, le capteur (240 ; 440) étant un capteur chauffable et le dispositif d'encapsulation (201 ; 300) étant configuré pour distribuer et évacuer une chaleur délivrée par le capteur (240 ; 440) chauffable.

14. Système capteur (200 ; 400) selon la revendication 13, le capteur (240 ; 440) chauffable possédant une puissance électrique qui est supérieure à une puissance limite pour la sécurité intrinsèque en tant que mode de protection « e » en référence au capteur (240 ; 440) chauffable et la température de surface la plus élevée au niveau du côté extérieur du dispositif d'encapsulation (201 ; 300) étant inférieure à une température limite pour la protection antidéflagrante en référence au capteur (240 ; 440) chauffable.

15. Procédé (500) pour faire fonctionner un capteur chauffable, notamment dans une atmosphère explosible, comprenant :
- fourniture (510), dans un volume de mesure, d'un dispositif d'encapsulation pour un capteur chauffable selon l'une des revendications 1 à 9 et d'un capteur chauffable qui est disposé dans l'espace d'accueil du dispositif d'encapsulation, le dispositif d'encapsulation étant configuré pour distribuer et évacuer une chaleur délivrée par le capteur chauffable, et
- alimentation (520) du capteur chauffable avec une puissance électrique, la puissance électrique étant dimensionnée de telle sorte qu'elle est supérieure à une puissance limite pour la sécurité intrinsèque en tant que mode de protection « e » en référence au capteur chauffable.
